# FASCICULE DE BREVET EUROPEEN

(11) **EP 1 276 504 B1**
(45) Date de publication et mention de la délivrance du brevet: **26.01.2005**
(21) Numéro de dépôt: 01929754.8
(22) Date de dépôt: 27.04.2001
(51) Int. Cl.: A61K 45/06, A61P 35/00

(54) **MEDICAMENTS DESTINES AU TRAITEMENT DES PATHOLOGIES TUMORALES CONTENANT LE COMPOSE RO5-4864 ET UN AGENT INDUCTEUR DE L'APOPTOSE**
ARZNEIMITTEL ZUR ANWENDUNG BEI TUMORKRANKHEITEN WELCHES RO5-4864 UND EIN APOPTOSEAUSLÖSER ENTHÄLT
MEDICINES FOR TREATING TUMORAL PATHOLOGIES CONTAINING THE RO5-4864 COMPOUND AND AN APOPTOSIS-INDUCING AGENT

(30) Priorité: 28.04.2000 FR 0005438
(43) Date de publication de la demande: 22.01.2003
(73) Titulaire: CENTRE NATIONAL DE LA RECHERCHE SCIENTIFIQUE (CNRS), 75794 Paris Cedex 16 (FR); Institut Curie, 75248 Paris Cedex 05 (FR)
(72) Inventeur: DECAUDIN, Didier, F-91370 Verrières-le-Buisson (FR); KROEMER, Guido, F-75014 Paris (FR); POUPON, Marie-France, F-94260 Fresnes (FR); NEMATI, Fariba, F-75018 Paris (FR); BEURDELEY-THOMAS, Arnaud, F-75015 Paris (FR)
(74) Mandataire: Grosset-Fournier, Chantal Catherine
(86) Numéro de dépôt international: PCT/FR2001/001322
(87) Numéro de publication internationale: WO 2001/082969

(56) Documents cités:
- YUTAKA TANIMOTO E.A.: "Benzodiazepine receptor agonists modulate thymocyte apoptosis through reduction of the mitochondrial transmembrane potential " JAPANESE JOURNAL OF PHARMACOLOGY, vol. 79, no. 2, 1999, pages 177-183, XP000957554

## Description

La présente invention a pour objet l'utilisation du composé Ro5-4864, et de composés dérivés de ce dernier répondant à la formule (I) ci-après, pour la préparation de médicaments destinés au traitement des pathologies tumorales.

Actuellement, la cancérothérapie consiste en l'utilisation de la radiothérapie, de la chimiothérapie, ou de leur combinaison. Parmi les produits utilisés en chimiothérapie, on trouve les composés inducteurs de l'apoptose, à savoir les composés ayant un effet de stimulation de la mort programmée des cellules. Ces méthodes ont des effets secondaires néfastes, et sont mal supportées par les patients.

L'utilisation d'agents pharmacologiques visant à augmenter la susceptibilité des cellules tumorales à l'induction de l'apoptose, serait très avantageuse dans la mesure où elle permettrait de réduire sensiblement la posologie des composés directement ou indirectement inducteurs d'apoptose utilisés en radio ou chimiothérapie.

A ce titre, le composé PK11195 de formule suivante 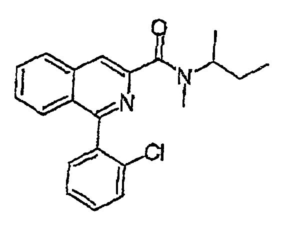 est décrit dans la demande internationale WO 99/66958 pour la préparation de médicaments destinés au traitement de cancers, en combinaison avec des inducteurs d'apoptose.

La présente invention découle de la mise en évidence sur un modèle expérimental animal par les Inventeurs du fait que le composé Ro5-4864, et les composés dérivés de ce dernier répondant à la formule (Ia) ci-après, ont un effet de stimulation de l'apoptose induite par les composés inducteurs d'apoptose, supérieur à celui observé avec le Diazépam et le composé PK11195 indiqué ci-dessus.

La présente invention a pour objet l'utilisation d'au moins un composé choisi parmi ceux de formule (I) suivante : 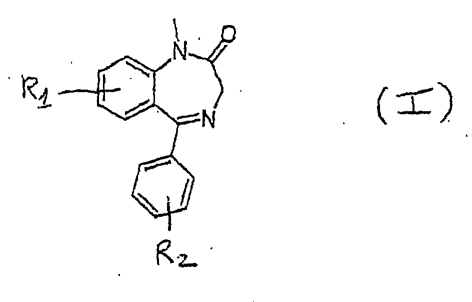 dans laquelle :
- R₁ représente un atome d'hydrogène, ou un atome d'halogène tel que Cl,
- R₂ un atome d'halogène tel que Cl,
pour la préparation d'un médicament destiné au traitement des pathologies tumorales, ledit composé étant avantageusement en association avec au moins un agent inducteur de l'apoptose, dans le cadre de produits de combinaison pour une utilisation simultanée, séparée ou étalée dans le temps, en thérapie cancéreuse.

L'invention a plus particulièrement pour objet l'utilisation susmentionnée, du composé Ro5-4864 de formule suivante :

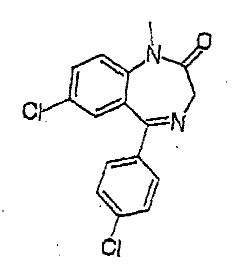

L'invention à plus particulièrement pour objet les produits comprenant :
* au moins un composé de formule (I) ci-dessus dans laquelle R₁ et R₂ représentent un atome d'halogène tel que Cl,
* et au moins un agent inducteur de l'apoptose, comme produits de combinaison pour une utilisation simultanée, séparée ou étalée dans le temps, en thérapie cancéreuse.

Des produits de combinaison définis ci-dessus préférés dans le cadre de la présente invention sont ceux comprenant à titre de composé de formule (I) le composé Ro5-4864 dont la formule est indiquée ci-dessus.

L'invention concerne également l'utilisation des produits de combinaison tels que définis ci-dessus, comprenant au moins un agent inducteur de l'apoptose, le cas échéant inséré dans un vecteur approprié pour la thérapie génique, notamment un vecteur d'origine viral, ledit agent étant choisi parmi ceux qui endommagent l'ADN, les ligands naturels ou synthétiques du récepteur aux glucocorticoïdes, ou autres composés pro-apoptotiques.

L'invention a plus particulièrement pour objet l'utilisation des produits de combinaison tels que définis ci-dessus, caractérisés en ce que l'agent inducteur de l'apoptose est choisi parmi :
- les dérivés des glucocorticoïdes, tels que la dexaméthasone,
- les agents alkylants tels que :
   . les moutardes à l'azote, notamment le cyclophosphamide,
   . les complexes de platine,
   . les dérivés de l'éthylène-imine,
   . les dérivés de diméthane sulfonoxy-alcanes,
   . les dérivés de la pipérazine,
- les inhibiteurs des topoisomérases tels que :
   . les inhibiteurs de la topoisomérase 2, notamment les anthracyclines, l'épipodophyllotoxine tel que l'étoposide,
   . les inhibiteurs de la topoisomérase 1, notamment les dérivés de la camptothecine,
- les antimétabolites tels que :
   . les antifolates, notamment le méthotrexate,
   . les antipurines, notamment la 6-mercaptopurine,
   . les antipyrimidines, notamment la 5-fluorouracile,
- les antimitotiques tels que :
   . les vinca-alcaloïdes,
   . les taxoïdes, notamment le taxol, le taxotere,
- les composés cytolytiques tels que :
   . la bléomycine,
   . la dacarbazine,
   . l'hydroxycarbamide,
   . l'asparaginase,
   . la mitoguazone,
   . la plicamycine,
- les radiations gamma,
- l'étoposide,
- la doxorubicine,
- des composés tels que la lonidamine ou dérivés,
- des anticorps monoclonaux ou autres, ou tout composé ou autre traitement d'immunothérapie.

De préférence, les produits de combinaison susmentionnés de l'invention, contiennent un produit de formule (I) dans laquelle R₁ et R₂ représentent un atome d'halogène tel que Cl, avantageusement le composé RO5-4864, et un agent inducteur d'apoptose dans un rapport en poids d'environ 1 : 5 à environ 1 : 1.

De préférence encore, les doses de RO5-4864 envisagées chez l'homme se situent entre 1 et 10 mg quotidiens.

Avantageusement, les produits de combinaison susmentionnés utilisés selon l'invention, comprennent aussi un ou plusieurs véhicules pharmaceutiquement acceptables, et se présentent sous une forme administrable par voie orale ou parentérale, notamment par voie intramusculaire, intraveineuse, ou sous-cutanée.

L'invention concerne également l'utilisation de produits de combinaison susmentionnés; pour la préparation d'un médicament destiné au traitement des pathologies tumorales telles que définies ci-dessus.

L'invention sera davantage illustrée à l'aide de la description qui suit de la mise en oeuvre d'expériences de mesure de l'effet du composé Ro5-4864 de stimulation de l'apoptose induite par les composés inducteurs d'apoptose.
- Matériel : souris nu/nu femelles, d'un poids de 30 g, âgées de 6-8 semaines. Élevage dans des conditions dépourvues d'agents pathogènes, sous lumière artificielle (12 heures de clarté, 12 heures d'obscurité).
- Greffe sous-cutanée interscapulaire des tumeurs humaines étudiées.
- Traitement des souris avec un composé inducteur d'apoptose tel que défini ci-dessus, et le composé Ro5-4864, lorsque les tumeurs, atteignent un diamètre d'environ 5 mm (ou un volume de 60 mm³).
- Pesée hebdomadaire des souris. Mesure 3 fois par semaine des tumeurs.
- Analyse : mesure du volume tumoral (V) et du volume tumoral relatif (VTR) :
- V = a² x b/2, a le plus grand diamètre et b le plus petit diamètre de la tumeur.
- VTR = Vx/Vi, Vx le volume moyen au temps x, Vi le volume moyen au temps JO.
- Comparaison des résultats obtenus chez les souris traitées par le composé inducteur d'apoptose seulement, avec les résultats obtenus d'une part chez les souris traitées par le composé inducteur d'apoptose et le composé Ro5-4864, et, d'autre part chez les souris traitées par le composé Ro5-4864 seul.
- Sacrifice des souris lorsque les tumeurs atteignent un volume de 2500 mm³.

### I- Introduction

L'apoptose , ou mort cellulaire programmée, est contrôlée au niveau d'un effecteur central commun localisé au niveau des mitochondries (Kroemer and Reed, 2000). La perméabilité membranaire mitochondriale (membrane externe) constitue l'événement irréversible qui induit la mort cellulaire. Cette perméabilité est sous contrôle, d'une part, d'un complexe multiprotéique permettant l'ouverture ou la fermeture de pores membranaires et, d'autre part, de protéines régulatrices comme Bc1-2 ou Bax.

Dans ce complexe multiprotéique, appelé pore de transition de perméabilité, se trouve le récepteur périphérique des benzodiazépines (RPB) (Zoratti and Szabo, 1995). Il existe deux sites de fixation des benzodiazépines : les récepteurs centraux localisés dans le SNC et au niveau de la membrane plasmique des cellules, et les récepteurs périphériques localisés principalement au niveau de la membrane externe mitochondriale. De même, il existe différents types de ligands des RPB : des ligands endogènes (DBI ou *diazepam binding inhibitor* et porphyrines) et des ligands exogènes (les Benzodiazépines comme le diazépam et le 4' chlorodiazépam (ou RO5-4864) et les carboxamides d'isoquinolines comme le PK11195).

Nous avons montré, sur différentes lignées cellulaires, que l'effet antitumoral des anticorps anti-Fas-récepteur (FasR) était augmenté par des ligands du RPB (RO5-4864, diazépam et PK11195) et que cet effet était nettement (au moins 3 ou 4 fois) supérieur avec le RO5-4864 qu'avec les autres composés. D'autre part, seul le RO5-4864 est capable, sur une lignée transfectée par un gène de résistance à l'apoptose telle que les lignées surexprimant les gènes *bcl-2* ou *bcl*-*X*_{*L*}, d'augmenter l'effet apoptotique des anticorps anti-FasR. De même, sur deux tumeurs humaines de carcinome bronchique à petites cellules greffées chez la souris *:nude,* nous avons montré que le RO5-4864 augmentait l'effet de la chimiothérapie antitumorale.

### II- Matériel et méthodes :

### 1- Lignées cellulaires, conditions de culture et induction de l'apoptose :

Les lignées humaines Jurkat (lymhoïde T), SHEP (neuroblastome) transfecté avec un vecteur contrôle ou avec un vecteur *bcl-2* ou *bcl-X*_{*L*}_{.}, 143N2 (ostéosarcome) et SNB79 (glioblastome) ont été cultivées en DMEM ou RPMI, 1640 (Sigma Chemical C°, St Louis, MO) supplémenté en SVF 10% (Dutscher, Brumath, France), pénicilline G(10² UI/ml) + streptomycine (50 µg/ml) (Sigma) et L-glutamine (2nM, Sigma).

Les cellules ont été cultivées en présence d'anticorps anti-FasRécepteur CH11 (1 µg/ml ; Immunotech, Marseille, France) concomitamment ou après exposition au RO5-4864 (BioBlock Scientific, Illkirch, France), diazépam (Roche, Neuilly sur Seine, France), ou PK11195 (BioBlock), à différentes concentrations.

### 2- Quantification de l'apoptose et de la viabilité cellulaire :

Le fluorochrome lipophilique DiOC₆(3) (Molecular Probes, Eugene, OR) a été utilisé pour mesurer le protentiel membranaire des mitochondries (Δψₘ). Brièvement, les cellules ont été incubées 15 min à 37°C en présence de 40nM de DiOC₆(3), avec analyse immédiate de l'incorporation de la fluorescence sur un cytofluoromètre de type Epics Profile II (Coulter, Miami, FL). L'hydroéthydine (HE) (2 µM, 15 min à 37°C, Molecular Probes) a été utilisée pour mesurer la production d'anion superoxide (Marchetti et al., 1996). Enfin, la proportion de cellules ayant perdu une partie de l'ADN chromosomal (cellules subdiploïdes) a été déterminée par iodure de propidium sur cellules fixées en éthanol (Nicoletti et al, 1991).

La viabilité cellulaire a été mesurée par un test au bleu de méthylène (Dimanche-Boitrel et al., 1992).

### 3- Etudes in vivo :

Des souris suisses, nu/nu, femelles, âgées de 6 à 8 semaines et pesant environ 30g, ont été utilisées pour les expériences *in vivo*. Pour les essais thérapeutiques, les souris greffées avec les tumeurs humaines ont été randomisées en groupes équivalents de 4 à 8 animaux et traitées dès que la tumeur atteignait un diamètre de 5 mm (soit un volume approximatif de 60 mm³). La croissance tumorale a été évaluée par la mesure des deux diamètres tumoraux perpendiculaires.

Deux tumeurs humaines de carcinome bronchique à petites cellules ont été utilisées, la SCLC6 et la SCLC61.

La chimiothérapie, administrée par voie intrapéritonéale, a comporté, soit de l'étoposide seul à la dose de 12 mg/kg/j J1 à J3, soit une association étoposide 12 mg/kg/j + ifosfamide 90 mg/kg/j de J1 à J3. Le RO5-4864 a été préparé dans un excipient contenant éthanol + Tween et injecté par voie sous-cutanée à la dose 12 à 40 mg/kg/j de J1 à J3. Le groupe témoin a reçu des injections de sérum physiologique.

### 4- Analyses statistiques :

Un test student t a été utilisé pour comparer la croissance des tumeurs xénogreffées chez les souris *nude* des différents groupes randomisés.

### III- Résultats :

### 1- Etudes in vitro :

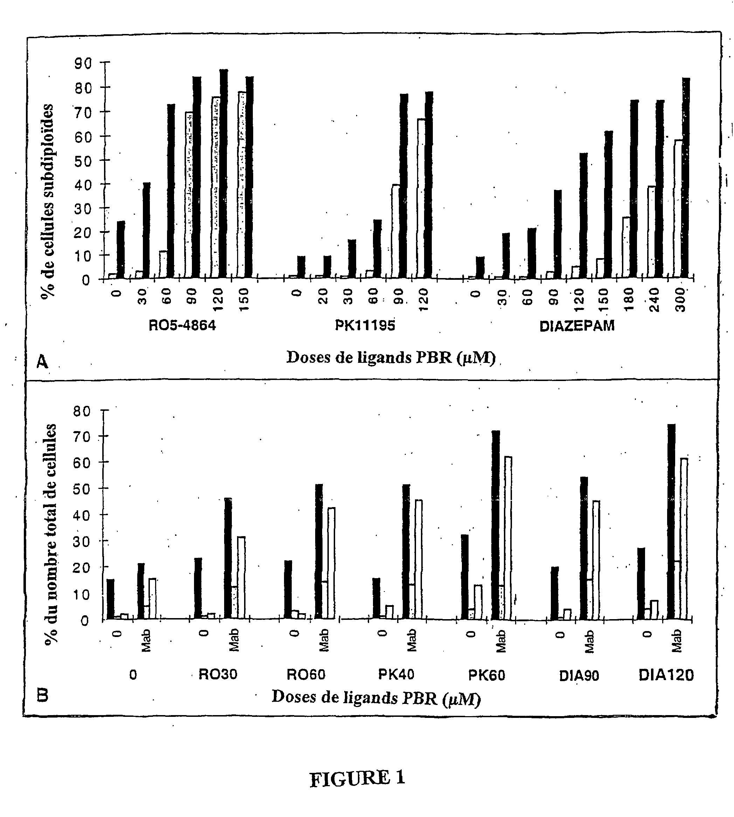

Sur la lignée lymphoïde T Jurkat, le RO5-4864, le diazépam et le PK11195 potentialisent l'effet apoptotique de l'anticorps anti-FasR CH11 (Fig. 1A et 1B). Cet effet est maximal avec le RO5-4864 puisque, à concentration équivalente de 60µM, la proportion de cellules subdiploïdes (apoptotiques) est de 73%, 25% et 20% pour le RO5-4864, le PK11195 et le diazépam, respectivement, en association avec le CH11.

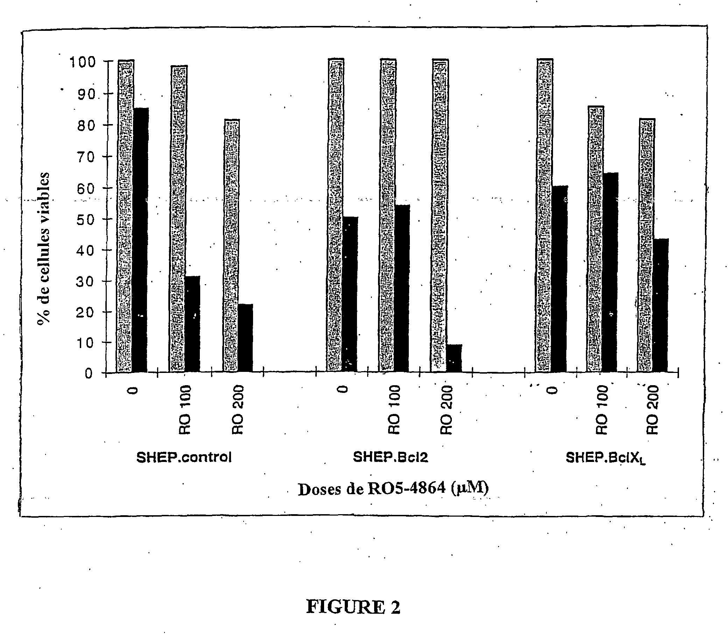

Le RO5-4864 réverse la résistance à l'anticorps anti-FasR CH11 des lignées SHEP-contrôle, SHEP-bcl-2, ou bcl-X_{L}, 143N2 et SNB79. Inversement, cette réversion n'est observée avec le diazépam et le PK11195 que sur la seule lignée SHEP-contrôle (fig. 2, 3 et 4).

### 2- Etudes in vivo :

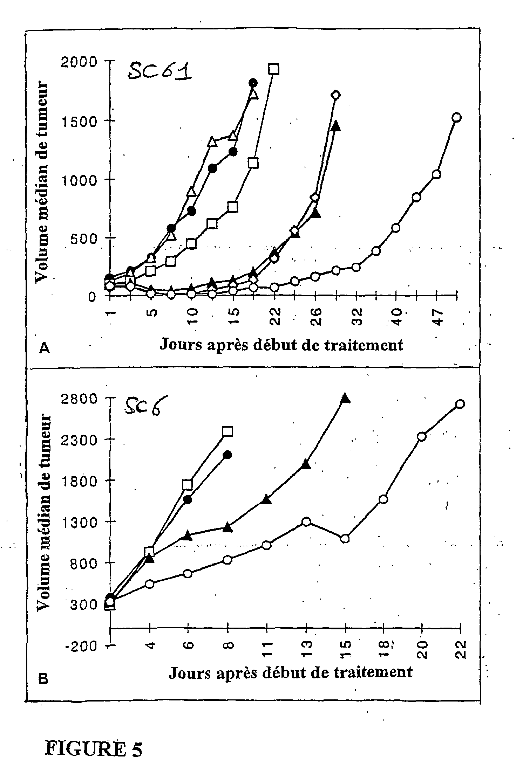

Les souris greffées avec la tumeur humaine SCLC61 ont été traitées par l'étoposide injecté par voie intrapéritonéale à la dose de 12 mg/kg/j de J1 à J3, avec ou sans RO5-4864 à la dose de 12 mg/kg/j SC J1 à J3. La croissance tumorale n'a pas été modifiée par le RO5-4864 seul. Par contre, l'effet antitumoral de l'étoposide est augmenté par l'administration concomitante de RO5-4864 (p<0.005) (fig. 5A). L'excipient du RO5-4864, injecté seul ou en association avec l'étoposide, ne modifie pas la croissance tumorale du groupe contrôle et du groupe étoposide.

Les tumeurs SCLC6 greffées ont été traitées par l'association étoposide 12 mg/kg/j+ifosfamide 90 mg/kg/j de J1 à J3, avec ou sans RO5-4864 40 mg/kg/j de J1 à J3. L'effet antitumoral de la chimiothérapie a été augmenté par le RO5-4864 (p<0.05) (fig. 5B).

### IV- Conclusion

L'ensemble de ces expériences montre que le RO5-4864, sur plusieurs variétés de lignées humaines, est capable de lever la résistance aux anticorps anti-FasR et, sur 2 tumeurs de carcinome bronchique à petites cellules, d'augmenter l'effet antitumoral d'une chimiothérapie associant étoposide +/- ifosfamide. De plus, sur la lignée de neuroblastome humain SHEP transfectée par les gènes *bcl-2* ou *bcl-X*_{*L*}_{,}, le RO5-4864 réverse la résistance aux anticorps anti-FasR.

Différentes expériences ont montré que l'effet du RO5-4864 était nettement supérieur à celui du diazépam ou du PK11195 et que, en particulier, le RO5-4864 était le seul capable de réverser la résistance aux anticorps anti-FasR sur la lignée SHEP transfectée par les gènes *bcl-2* ou *bcl*-*X*_{*L*}_{,}.

### V- Références :

Kroemer G, Reed JC. Mitochondrial control of cell death. Nat Med 2000 ;6 :513-19.

Zoratti M, Szabo I. The mitochondrial permeability transition. Biochem Biophys Acta Rev Biomembr 1995 ;1241 : 139-76.

Marchetti P, Hirsch T, Zamzami N, Castedo M, Decaudin D, Susin SA, et al. Mitochondrial permeability transition triggers lymphocyte apoptosis. J Immunol 1996 ; 157 :4830-6.

Nicoletti I, Migliorati G, Plagliacci MC, Riccardi C. A rapid simple method for measuring thymocyte apoptosis by propidium iodide staining and flow cytometry. J Immunol Methods 1991 ;139 :271-80.

Dimanche-Boitrel M-T, Pelletier H, Genne P, Petit J-M, Le Grimellec C, Canal P, et al. Confluence-dependent resistance in human colon cancer cells : role of reduced drug accumulation and low intrinsic chemosensitivity of resting cells. Int. J. Cancer 1992 ; 50 :677-82.

### VI- Légendes des figures :

1. Figure 1 : culture des cellules Jurkat en présence de CH11 avec (histogrammes blancs) ou sans (histogrammes noirs) RO5-4864, PK11195 ou diazépam. En A, détermination de la proportion de cellules subdiploïdes (en ordonnée), en fonction de la concentration en µM des ligands PBR RO5-4864, PK11195 et DIAZEPAM. En B, détermination du % de cellules (en ordonnée), avec chute du (Δψm) (histogrammes noirs), production d'anion superoxide (histogrammes gris) et subdiploïdes (histogrammes blancs), en absence (o) ou en présence d'anticorps antiFas CH11 (encore désigné Mab); en fonction de la concentration en µM des ligands PBR RO5-4864 (à 30µM ou RO30, et à 60 µM ou RO60), PK11195 (à 40µM ou PK40, et à 60 µM ou PK60) et DIAZEPAM (à 90µM ou DIA90, et à 120µM ou DIA120).
2. Figure 2 : culture des lignées SHEP de contrôle (SHEP.control), des lignées SHEP transfectées par bcl2 (SHEP.Bcl2), et des lignées SHEP transfectées par bclX_{L} (SHEP.BclX_{L}), en présence de CH11 avec (histogrammes noirs) ou sans (histogrammes gris) RO5-4864. Evaluation de la viabilité cellulaire (test au bleu de méthylène; pourcentage de cellules en ordonnée), en fonction de la concentration de RO5-4864 (0, 100µM ou RO 100, 200µM ou RO 200).

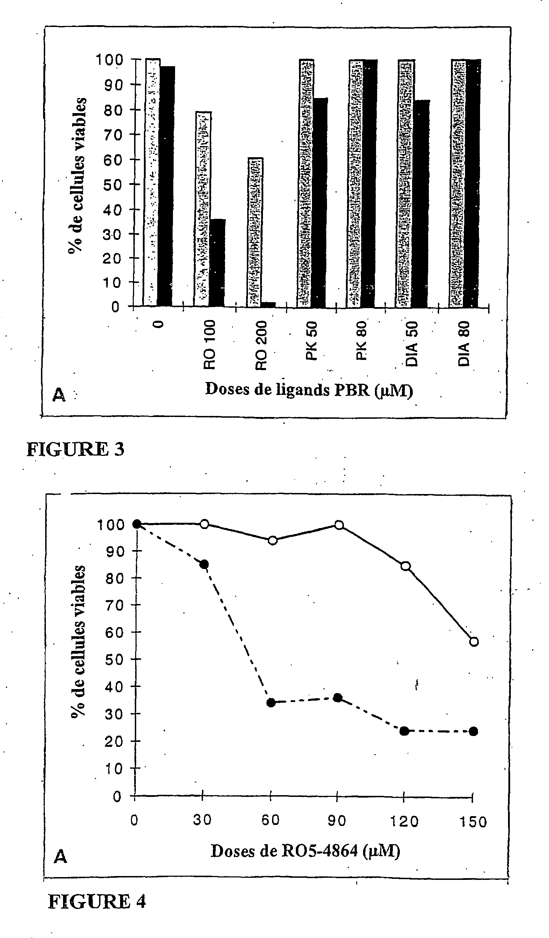

3. Figure 3 : culture de la lignée 143N2 en présence de CH11 avec (histogrammes noirs) ou sans (histogrammes gris) RO5-4864, PK11195 ou diazépam. Évaluation de la viabilité cellulaire (test au bleu de méthylène; pourcentage de cellules en ordonnée), en fonction de la concentration de RO5-4864 (100µM ou RO 100, 200µM ou RO 200), PK11195 (50µM ou PK50, 80 µM ou PK80) et DIAZEPAM (50µM ou DIA50, 80µM ou DIA80).
4. Figure 4 : culture de la lignée SNB79 en présence de CH11 avec (●) ou sans (○) RO5-4684. Évaluation de la viabilité cellulaire (test au bleu de méthylène; pourcentage de cellules en ordonnée), en fonction de la concentration (µM) de RO5-4864.
5. Figure 5 : en A, tumeur SCLC61 xenogreffée chez la souris *nude* et traitée par étoposide avec (○) ou sans (▲) RO5-4684. Deux groupes contrôles ont reçu l'excipient du RO5-4864 seul (Δ) ou avec étoposide (◇). En B, tumeur SCLC6 xenogreffée chez la souris nude et traitée par étoposide + ifosfamide avec (○) ou sans (▲) RO5-4684. Le volume médian des tumeurs est indiqué en ordonnée en fonction du nombre de jours après le début du traitement. Deux groupes contrôles ont reçu des injections soit de RO-4864 seul (□), soit de NaCl 0,9% (●).

## Revendications

1. Utilisation de composés de formule (I) suivante : 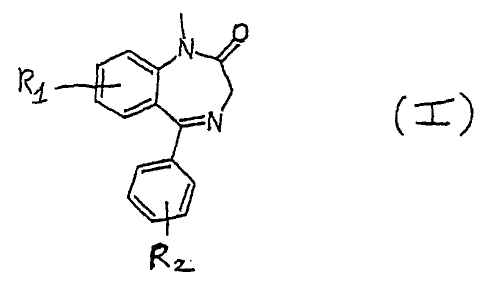 dans laquelle R₁ et R₂ représentent un atome d'halogène tel que Cl,
pour la fabrication d'un médicament destiné à augmenter la susceptibilité des cellules tumorales à l'induction de l'apoptose par des agents inducteurs d'apoptose, dans le traitement des pathologies tumorales.

2. Utilisation selon la revendication 1, pour la préparation de médicaments sous forme de produits de combinaison comprenant au moins un composé de formule (I), et au moins un agent inducteur de l'apoptose, pour une utilisation simultanée, séparée ou étalée dans le temps desdits produits en thérapie cancéreuse.

3. Utilisation selon la revendication 1 ou 2, **caractérisée en ce que** le composé de formule (I) est le composé Ro5-4864 de formule suivante :

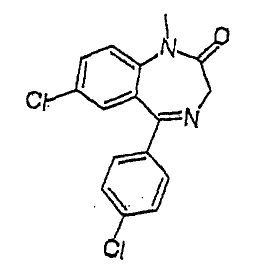

4. Utilisation selon l'une des revendications 1 à 3, **caractérisée en ce que** le médicament se présente sous une forme administrable par voie orale ou parentérale, notamment par voie intramusculaire ou intraveineuse.

5. Utilisation selon l'une des revendications 1 à 4, **caractérisée en ce que** les produits de combinaison comprennent au moins un agent inducteur de l'apoptose, le cas échéant inséré dans un vecteur approprié pour la thérapie génique, notamment un vecteur d'origine viral, ledit agent étant choisi parmi ceux qui endommagent l'ADN, les ligands naturels ou synthétiques du récepteur aux glucocorticoïdes, ou autres composés pro-apoptotiques.

6. Utilisation selon l'une des revendications 1 à 5, **caractérisée en ce que** l'agent inducteur de l'apoptose est choisi parmi :
- les dérivés des glucocorticoïdes, tels que la dexaméthasone,
- les agents alkylants tels que :
. les moutardes à l'azote, notamment le cyclophosphamide,
. les complexes de platine,
. les dérivés de l'éthylène-imine,
. les dérivés de diméthane sulfonoxy-alcanes,
. les dérivés de la pipérazine,
- les inhibiteurs des topoisomérases tels que :
. les inhibiteurs de la topoisomérase 2, notamment les anthracyclines, l'épipodophyllotoxine tel que l'étoposide,
. les inhibiteurs de la topoisomérase 1, notamment les dérivés de la camptothecine,
- les antimétabolites tels que :
. les antifolates, notamment le méthotrexate,
. les antipurines, notamment la 6-mercaptopurine,
. les antipyrimidines, notamment la 5-fluorouracile,
- les antimitotiques tels que :
. les vinca-alcaloïdes,
. les taxoïdes, notamment le taxol, le taxotere,
- les composés cytolytiques tels que :
. la bléomycine,
. la dacarbazine,
. l'hydroxycarbamide,
. l'asparaginase,
. la mitoguazone,
. la plicamycine,
- des composés tels que la lonidamine ou dérivés,
- ou des anticorps monoclonaux ou autres, ou tout composé ou autre traitement d'immunothérapie.

7. Utilisation selon l'une des revendications 1 à 6, **caractérisé en ce que** les produits de combinaison comprennent au moins un agent inducteur de l'apoptose choisi parmi :
- les radiations gamma,
- l'étoposide,
- la doxorubicine,
- la dexamethasone,
- la lonidamine.

8. Utilisation selon l'une des revendications 1 à 7, **caractérisés en ce que** les produits de combinaison contiennent un produit de formule (I) et un agent inducteur d'apoptose dans un rapport en poids d'environ 1 : 5 à environ 1 : 1.

9. Utilisation selon l'une des revendications 1 à 8, **caractérisés en ce que** les produits de combinaison comprennent aussi un ou plusieurs véhicules pharmaceutiquement acceptables.

10. Produits de combinaison pour une utilisation simultanée, séparée ou étalée dans le temps, en thérapie cancéreuse, **caractérisés en ce qu'**ils contiennent un produit de formule (I) défini dans la revendication 1 ou 3, et un agent inducteur d'apoptose tel que défini dans la revendication 6 ou7, dans un rapport en poids d'environ 1 : 5 à environ 1 : 1.

## Claims

1. The use of compounds of the following formula (I) : 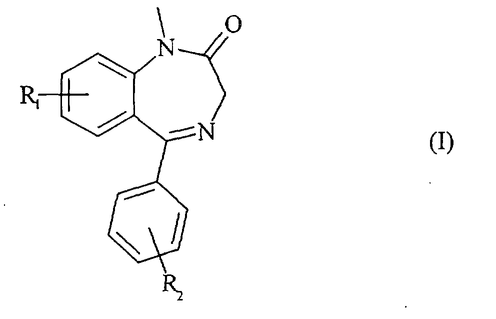 in which R₁ and R₂ represent a halogen atom such as Cl,
for the manufacture of a medicament for increasing the sensitivity of tumoral cells to induction of apoptosis by apoptosis-inducing agents in the treatment of tumoral pathologies.

2. The use according to Claim. 1 for the manufacture of medicaments in the form of combination products comprising at least one compound of formula (I) and at least one apoptosis-inducing agent, for a simultaneous, separate or spread over time use of said products in cancer therapy.

3. The use according to Claim 1 or 2, **characterized in that** the compound of formula (I) is the compound Ro5-4864 of the following formula :

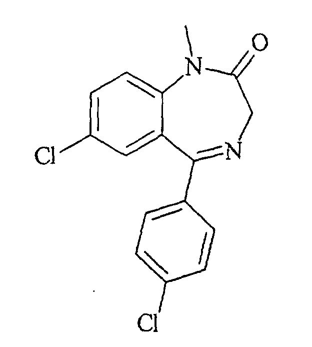

4. The use according to any one of Claims 1 to 3, **characterized in that** the medicament is in a form that can be administered orally or parenterally, especially by intramuscular or intravenous route.

5. The use according to any one of Claims 1 to 4, **characterized in that** the combination products comprise at least one apoptosis-inducing agent, if necessary inserted in a vector suitable for gene therapy, especially a vector of viral origin, said agent being selected among those that damage DNA, natural or synthetic ligands of the receptor to glucocorticoids, or other pro-apoptotic compounds.

6. The use according to one of Claims 1 to 5, **characterized in that** the apoptosisinducing agent is selected from :
- derivatives of glucocorticoids, such as dexamethasone,
- alkylating agents such as:
. nitrogen mustards, especially cyclophosphamide,
. platinum complexes,
. derivatives of ethylene-imine,
. derivatives of dimethane sulphonoxy-alkanes,
. derivatives of piperazine,
- inhibitors of topoisomerases such as:
. inhibitors of topoisomerase 2, especially anthracyclines, epipodophyllotoxin such as etoposide,
. inhibitors of topoisomerase 1, especially derivatives of camptothecine,
- antimetabolites such as:
. antifolates, especially methotrexate,
. antipurines, especially 6-mercaptopurine,
. antipyrimidines, especially 5-fluorouracil,
- antimitotics such as:
. vinca alkaloids,
. taxoids, especially taxol, taxotere,
- cytolytic compounds such as:
. bleomycin,
. dacarbazine,
. hydroxycarbamide,
. asparaginase,
. mitoguazone,
. plicamycin,
- compounds such as lonidamine or derivatives,
- or monoclonal or other antibodies, or any compound or other immunotherapy treatment.

7. The use according to one of Claims 1 to 6, **characterized in that** the combination products comprise at least one apoptosis-inducing agent selected from :
- gamma rays,
- etoposide,
- doxorubicin,
- dexamethasone,
- lonidamine.

8. The use according to one of Claims 1 to 7, **characterized in that** the combination products contain a product of formula (I) and an apoptosis-inducing agent in a weight ratio of about 1:5 to about 1:1

9. The use according to one of Claims 1 to 8, **characterized in that** the combination products also comprise one or several pharmaceutically acceptable vehicles.

10. Combination products for a simultaneous, separate or spread over time use in cancer therapy, **characterized in that** they contain a product of formula (I) defined in Claim 1 or 3 and an apoptosis-inducing agent such as defined in Claim 6 or 7 in a weight ratio of about 1:5 to about 1:1.

## Patentansprüche

1. Verwendung von Verbindungen der folgenden Formel (I): 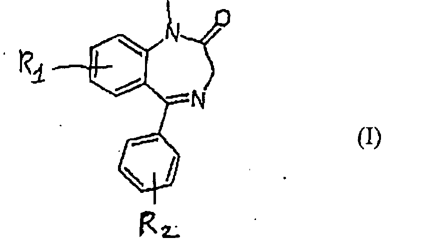 in der R₁ und R₂ ein Halogenatom, wie Cl, bedeuten,
für die Herstellung eines Medikaments zur Erhöhung der Empfindlichkeit von Tumorzellen gegenüber der Induktion von Apoptose durch Apoptose-induzierende Mittel bei der Behandlung von Tumorerkrankungen.

2. Verwendung nach Anspruch 1 für die Herstellung von Medikamenten in Form von Kombinationsprodukten, die wenigstens eine Verbindung der Formel (I) und wenigstens ein Apoptose-induzierendes Mittel enthalten, für eine gleichzeitige, getrennte oder zeitlich gestaffelte Verwendung der Produkte in der Krebstherapie.

3. Verwendung nach Anspruch 1 oder 2, **dadurch gekennzeichnet, dass** die Verbindung der Formel (I) die Verbindung Ro5-4864 der folgenden Formel ist:

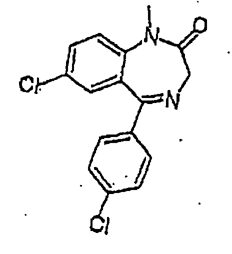

4. Verwendung nach einem der Ansprüche 1 bis 3, **dadurch gekennzeichnet, dass** das Medikament in einer Form vorliegt, die oral oder parenteral verabreicht werden kann, insbesondere intramuskulär oder intravenös.

5. Verwendung nach einem der Ansprüche 1 bis 4, **dadurch gekennzeichnet, dass** die Kombinationsprodukte wenigstens ein Apoptose-induzierendes Mittel aufweisen, gegebenenfalls in einen für Gentherapie geeigneten Vektor inseriert, insbesondere einen Vektor viraler Herkunft, wobei das Mittel aus jenen, die die DNA schädigen, natürlichen oder synthetischen Liganden des Glucocorticoid-Rezeptors oder anderen proapoptotischen Verbindungen ausgewählt wird.

6. Verwendung nach einem der Ansprüche 1 bis 5, **dadurch gekennzeichnet, dass** das Apoptose-induzierende Mittel ausgewählt wird aus:
- Glucocorticoid-Derivaten, wie Dexamethason,
- Alkylantien, wie:
- Stickstofflostverbindungen, insbesondere Cyclophosphamid,
- Platinkomplexen,
- Ethylenimin-Derivaten,
- Derivaten der Dimethansulfonoxyalkane,
- Piperazin-Derivaten,
- Topoisomerase-Inhibitoren, wie:
- Topoisomerase-2-Inhibitoren, insbesondere Anthracycline, Epipodophyllotoxin, wie Etoposid,
- Topoisomerase-1-Inhibitoren, insbesondere Derivate von Camptothecin,
- Antimetaboliten, wie:
- Antifolaten, insbesondere Methotrexat,
- Antipurinen, insbesondere 6-Mercaptopurin,
- Antipyrimidinen, insbesondere 5-Fluorouracil,
- Antimitotika, wie:
- Vinca-Alkaloide,
- Taxoide, insbesondere Taxol, Taxoter,
- cytolytischen Verbindungen, wie:
- Bleomycin,
- Dacarbazin,
- Hydroxycarbamid,
- Asparaginase,
- Mitoguazon,
- Plicamycin,
- Verbindungen wie Lonidamin oder Derivate,
- oder monoklonalen oder anderen Antikörpern, oder jeder Verbindung oder anderen Behandlung der Immuntherapie.

7. Verwendung nach einem der Ansprüche 1 bis 6, **dadurch gekennzeichnet, dass** die Kombinationsprodukte wenigstens ein Apoptose-induzierendes Mittel, ausgewählt aus
- Gammastrahlen,
- Etoposid,
- Doxorubicin,
- Dexamethason,
- Lonidamin,
umfassen.

8. Verwendung nach einem der Ansprüche 1 bis 7, **dadurch gekennzeichnet, dass** die Kombinationsprodukte ein Produkt der Formel (I) und ein Apoptose-induzierendes Mittel in einem Gewichtsverhältnis von etwa 1:5 bis etwa 1:1 enthalten.

9. Verwendung nach einem der Ansprüche 1 bis 8, **dadurch gekennzeichnet, dass** die Kombinationsprodukte auch einen oder mehrere pharmazeutisch unbedenkliche Träger enthalten.

10. Kombinationsprodukte für die gleichzeitige, getrennte oder zeitlich gestaffelte Verwendung in der Krebstherapie, **dadurch gekennzeichnet, dass** sie ein Produkt der Formel (I), wie in Anspruch 1 oder 3 definiert, und ein Apoptose-induzierendes Mittel, wie in Anspruch 6 oder 7 definiert, in einem Gewichtsverhältnis von etwa 1:5 bis etwa 1:1 enthalten.
